# EUROPÄISCHE PATENTSCHRIFT

(11) **EP 0 584 491 B1**
(45) Veröffentlichungstag und Bekanntmachung des Hinweises auf die Patenterteilung: **03.05.1995**
(21) Anmeldenummer: 93110424.4
(22) Anmeldetag: 30.06.1993
(51) Int. Cl.: C07D 213/61, C07D 213/64, C07D 213/73

(54) **Verfahren zur Herstellung von 2,5-disubstituierten Pyridinen**
Process for the production of 2,5-disubstituted pyridines
Procédé pour la production de pyridines 2,5-disubstitués

(30) Priorität: 13.07.1992 DE 4223013
(43) Veröffentlichungstag der Anmeldung: 02.03.1994
(73) Patentinhaber: BAYER AG, 51368 Leverkusen (DE)
(72) Erfinder: Kraus, Helmut, Dr., D-51519 Odenthal (DE)

(56) Entgegenhaltungen:
- EP-A- 0 060 071
- DD-A- 240 010
- COLLECTION CZECHOSLOVAK CHEM. COMMUN. Bd. 48, Nr. 11, 1983, Seiten 3123 - 3129 S. MARCHALIN, J. KUTHAN 'The use of 4-R(1) -benzylidene-4-phenylbenzoylacetonitriles for synthesis of unsymmetrical substituted 3,5-dicyano-1,4-dihydropyridines and 2-amino-4H-pyrans'
- ANGEW. CHEM. INTERNAT. EDIT. ENGL. Bd. 8, Nr. 6, 1969, Seiten 458 - 459 H.U. SIEVEKING, W. L]TTKE 'Preparation of Enamino Dinitriles and Pyridines by Reduction of Geminal Dinitriles with LiAlH4'
- CHEM. PHARM. BULL. Bd. 32, Nr. 7, 1984, Seiten 2821 - 2824 Y. AKIYAMA ET AL. 'Studies on Conjugated Nitriles. V. Reaction of 3-Benzoyl- and 3-Ethoxycarbonylacrylonitriles with Enamines'
- SCIENCE Bd. 166, Nr. 3906, 1969, Seiten 765 - 766 J.P. FERRIS ET AL. 'Photochemical reactions and the chemical evolution of purines and nicotinamide derivatives'

## Beschreibung

Die Erfindung betrifft ein Verfahren zur Herstellung von 2,5-disubstituierten Pyridinen durch Umsetzung von Enaminen mit β-Amino-acrylnitrilen und anschließender Cyclisierung des offenkettigen Zwischenprodukts mit Protonensäuren oder Ammoniak.

2,5-disubstituierte Pyridine sind wichtige Zwischenprodukte zur Herstellung von Herbiziden (EP 108 483), Insektiziden (EP 235 725) und Pharmazeutika (CA 1.189.509, äquivalent zu DE-OS 2 812 585 und DE-OS 2 812 586).

Die benötigten 2,5-disubstituierten Pyridine können entweder durch Ringschlußreaktionen oder durch Substitution von 3-Alkyl-pyridinen erhalten werden Letztere Synthese ist jedoch mit einem Zwangsanfall an Stellungsisomeren behaftet.

Ein interessanter Aufbau ist die Ringverknüpfung von Morpholinopropen und Acrylsäurederivaten, also eine Reaktion von C₂- und C₃-Bausteinen. Die hierbei erhältlichen Dihydropyridine müssen allerdings aufwendig aromatisiert werden (EP 108 483). Die Verwendung von α-Chlor-acrylnitril (EP 162 464) führt in geringer Ausbeute direkt zum 2-Chlor-5-methyl-pyridin.

Die Umsetzung von C₄- und höheren Enaminen mit α-Chlor-acrylnitril ist in Tetrahedron 24 (1968), 3369, beschrieben. Ganz offensichtlich treten jedoch Nebenreaktionen auf; nur bei sehr tiefen Temperaturen, die technisch nur aufwendig realisierbar sind, ist eine befriedigende Ausbeute erhältlich (Synthesis 1985, 1116). Bei Betrachtung dieser Literaturstellen war es daher überraschend, daß bei Verwendung von β-Amino-acrylnitrilen in einfacher Weise die offenkettigen Zwischenprodukte der 4-R¹-5-amino-penta-2,4-dien-nitrile erhältlich sind, die zu den entsprechenden 2,5-disubstituierten Pyridinen cyclisiert werden können.

Die Erfindung betrifft ein Verfahren zur Herstellung von 2,5-disubstituierten Pyridinen der Formel 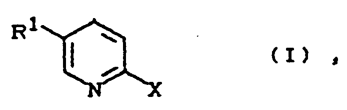
das gekennzeichnet ist durch die Umsetzung von 0,2-5 Mol, bevorzugt 0,4-3 Mol, besonders bevorzugt 0,7-1,5 Mol eines Enamins der Formel 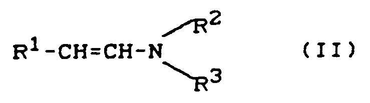
mit 1 Mol eines β-Amino-acrylnitrils der Formel 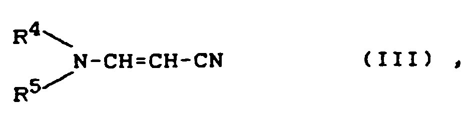
wobei in den Formeln
- R¹, R², R³, R⁴ und R⁵: unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ bzw. R⁴ und R⁵ jeweils gemeinsam, jedoch unabhängig voneinander, mit dem N-Atom, das sie substituieren, einen 5- bis 8-gliedrigen Ring bilden können, der ein weiteres Heteroatom aus der Gruppe von N, O und S enthalten kann, und
- X: für Chlor, Brom, Hydroxy oder Amino steht,
in einem Temperaturbereich von -70°C bis +50°C, bevorzugt -40°C bis +25°C, in flüssiger Phase und in Gegenwart von 0,7-20 Äquivalenten, bevorzugt 1-10 Äquivalenten, einer C₁-C₈-Carbonsäure zu einem offenkettigen Zwischenprodukt und anschließender Ringschlußreaktion mit 0,7-12 Äquivalenten, bevorzugt 1-6 Äquivalenten, einer Protonensäure oder von Ammoniak in einem Reaktionsmedium aus C₁-C₁₀-Carbonsäuren, halogenierten Kohlenwasserstoffen, Alkoholen, Amiden oder Gemischen daraus.

Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Octyle, bevorzugt die genannten C₁-C₄-Alkylreste.

Geradkettiges oder verzweigtes C₂-C₈-Alkenyl ist beispielsweise Vinyl, Propinyl, Allyl, die isomeren Butenyle, Pentenyle, Hexenyle oder Octenyle, bevorzugt die genannten C₃-C₄-Alkenylreste.

Geradkettiges oder verzweigtes C₂-C₈-Alkoxyalkyl ist beispielsweise Methoxymethyl, Ethoxymethyl sowie weitere Reste aus der Gruppe C₃-C₉-Alkyl, in welchem eine CH₂-Gruppe durch ein O-Atom ersetzt ist.

Geradkettiges oder verzweigtes C₃-C₈-Alkoxyalkenyl ist beispielsweise Methoxyvinyl, Ethoxyvinyl, Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, das am N-Atom durch C₁-C₄-Alkyl oder durch Hydroxy-C₁-C₄-alkyl substituiert sein kann, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können. In bevorzugter Weise seien als solche gesättigte oder ungesättigte heterocyclische Ringe Morpholin, Pyrrolidin und Piperidin, das durch C₁-C₄-Alkyl oder durch Hydroxy-C₁-C₄-alkyl substituiert sein kann, genannt.

Weiterhin können R² und R³ bzw. R⁴ und R⁵ jeweils gemeinsam, jedoch unabhängig voneinander, mit dem N-Atom, das sie substituieren, einen 5- bis 8-gliedrigen gesättigten oder ungesättigten Ring bilden, der ein weiteres Heteroatom aus der Gruppe N, O und S enthalten kann. Solche Ringe sind beispielsweise die oben genannten Heterocyclen.

Die Reaktion des erfindungsgemäßen Verfahrens kann für den Fall, daß R¹ Methyl ist, R² und R³ gemeinsam mit dem N-Atom, das sie substituieren, Morpholin darstellen und R⁴ und R⁵ Methyl bedeuten, formelmäßig wie folgt dargestellt werden: 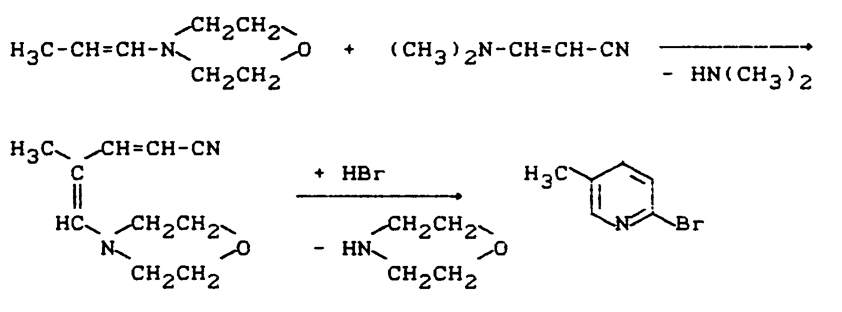
Aus dem Enamin und dem β-Amino-acrylnitril bildet sich demnach durch Austritt von Dimethylamin das formelmäßig gezeigte 4-R¹-5-amino-penta-2,4-dien-nitril, das im angegebenen Beispiel durch Behandlung mit Bromwasserstoff und unter Austritt von Morpholin zum 2-Brom-5-methyl-pyridin cyclisiert wird.

Im Rahmen der erfindungsgemäßen Umsetzung kann zwischen dem Enamin und dem β-Amino-acrylnitril ein Aminaustausch zustande kommen. Dies führt dazu, daß in der 1. Reaktionsstufe, die zu dem offenkettigen Zwischenprodukt führt, statt des Dimethylamins Morpholin eliminiert wird. Folgerichtig wird in der Ringschlußreaktion statt des Morpholins Dimethylamin eliminiert. Dieser Vorgang läßt sich formelmäßig wie folgt darstellen: 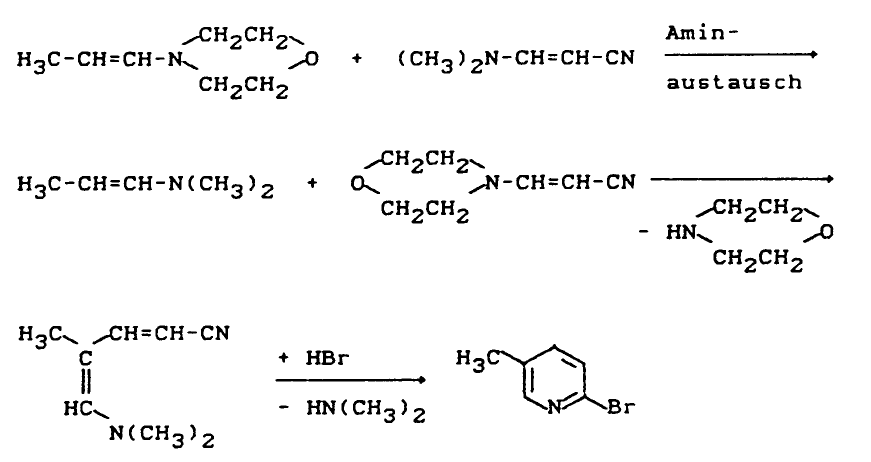
In der Praxis werden beide Formen der Reaktion, also ohne Aminaustausch und mit Aminaustausch, nebeneinander vorkommen. Dies ist jedoch für die zweistufige erfindungsgemäße Reaktion ohne Belang, da letztlich immer beide Aminogruppen eliminiert werden.

In bevorzugter Form werden Enamine eingesetzt, in denen an die Stelle von R¹ der Substituent R¹¹ tritt, der geradkettiges oder verzweigtes C₁-C₈-Alkyl, Cyclopropyl, Cyclopentyl, Cyclohexyl, Phenyl oder Benzyl bedeutet.

In besonders bevorzugter Weise werden Enamine eingesetzt, in denen an die Stelle von R¹¹ der Substituent R²¹ tritt, der geradkettiges oder verzweigtes C₁-C₄-Alkyl bedeutet.

In weiterhin bevorzugter Weise werden im erfindungsgemäßen Verfahren Enamine und β-Amino-acrylnitrile eingesetzt, in denen an die Stelle von R², R³, R⁴ und R⁵ die Substituenten R¹², R¹³, R¹⁴ und R¹⁵ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ bzw. R¹⁴ und R¹⁵ jeweils gemeinsam, jedoch unabhängig voneinander, mit dem N-Atom, das sie substituieren, einen 5- bis 8-gliedrigen Ring bilden können, der ein weiteres Heteroatom aus der Gruppe von N, O und S enthalten kann.

In weiterhin besonders bevorzugter Weise werden im erfindungsgemäßen Verfahren Enamine und β-Amino-acrylnitrile eingesetzt, in denen an die Stelle von R¹², R¹³, R¹⁴ und R¹⁵ die Substituenten R²², R²³, R²⁴ und R²⁵ treten, die unabhängig voneinander C₁-C₄-Alkyl bedeuten, wobei weiterhin R²² und R²³ bzw. R²⁴ und R²⁵ jeweils gemeinsam, jedoch unabhängig voneinander, mit dem N-Atom, das sie substituierten, Morpholin, Pyrrolidin oder Piperidin, das durch C₁-C₄-Alkyl oder durch Hydroxy-C₁-C₄-alkyl substituiert sein kann, bedeuten.

Enamine für das erfindungsgemäße Verfahren können in grundsätzlich bekannter Weise aus den zugrundeliegenden Aldehyden und den zugrundeliegenden sekundären Aminen hergestellt werden. Herstellungsbedingt kann ein solches Enamin auch mit einem Anteil des entsprechenden Aminals vergesellschaftet sein. Dies ist für den Erfolg des erfindungsgemäßen Verfahrens ohne Bedeutung.

β-Amino-acrylnitrile sind beispielsweise durch Umsetzung von Salzen des Formylacetonitrils mit sekundären Aminen (EP 18 473) oder durch Kondensation von ortho- Amiden mit Acetonitril leicht zugänglich.

Die Umsetzung im erfindungsgemäßen Verfahren erfolgt in beiden Stufen in flüssiger Phase. Sofern das eingesetzte Enamin und das β-Amino-acrylnitril gemeinsam eine flüssige Phase bilden, kann auf ein Lösungsmittel verzichtet werden. Ansonsten kann in einem Lösungsmittel aus der Gruppe der halogenierten Kohlenwasserstoffe, der Ketone, der Nitrile, der Amide, der Ester oder der Ether oder in einem Gemisch mehrerer von ihnen gearbeitet werden. Beispiele für solche Lösungsmittel sind: Methylenchlorid, Chloroform, Dichlorethan, Aceton, Methylethylketon, Acetonitril, Dimethylformamid, N-Methyl-pyrrolidon (NMP), N-Methyl-caprolactam (NMC), Tetramethylharnstoff, Essigsäuremethylester, Essigsäurebutylester, Methyl-tert.-butylether, tert.-Amyl-methylether, Tetrahydrofuran, Dioxan, bevorzugt ein oder mehrere halogenierte Kohlenwasserstoffe. Die Menge des eingesetzten Lösungsmittels beträgt 0 bis 2000 Gew.-%, bezogen auf die Gesamtmenge an Enamin und β-Amino-acrylnitril, bevorzugt 50 bis 2000 Gew.-%, besonders bevorzugt 200 bis 1500 Gew.-%; die im allgemeinen Gewichtsprozentbereich genannte Untergrenze von Null bedeutet hierbei den Umsatz des Enamins mit dem β-Amino-acrylnitrils in flüssiger Phase, jedoch ohne Verwendung eines zusätzlichen Lösungsmittels der genannten Art.

Das Verhältnis der beiden Reaktionspartner Enamin und β-Amino-acrylnitril wird im obengenannten molaren Bereich gewählt. Der Idealwert des molaren Verhältnisses der beiden Reaktionspartner liegt in offenkundiger Weise in der Nähe des Wertes 1:1, jedoch wird man meist die teurere Komponente im Unterschuß einsetzen, um ihren möglichst vollständigen Umsatz zu fördern.

Es ist für den Erfolg des erfindungsgemäßen Verfahrens unerheblich, welche der Reaktionskomponenten Enamin und β-Amino-acrylnitril vorgelegt und welche nachgesetzt wird; grundsätzlich ist auch die simultane Dosierung beiden Komponenten möglich. Hierbei kann ein mitzuverwendendes Lösungsmittel sowohl gemeinsam mit einer oder beiden Reaktionskomponenten zugegeben werden oder aber simultan zur Zugabe einer oder beider Reaktionskomponenten zugegeben werden. Die Zugabe der Reaktionskomponenten erfolgt im obengenannten Temperaturbereich; die Reaktion ist schwach exotherm.

Die Umsetzung zum offenkettigen Zwischenprodukt erfolgt in Gegenwart einer C₁-C₈-Carbonsäure, beispielsweise in Gegenwart von Ameisensäure, Essigsäure, Propionsäure, Buttersäure, Valeriansäure, Capronsäure oder Caprylsäure. In bevorzugter Weise wird in Gegenwart von Essigsäure oder Propionsäure, in besonders bevorzugter Weise in Gegenwart von Essigsäure gearbeitet. Die Säure wird in einer Menge von 0,7 bis 20 Äquivalenten, bevorzugt 1 bis 10 Äquivalenten, bezogen auf die Molzahl der im Unterschuß eingesetzten Reaktionskomponente, eingesetzt. Die Carbonsäure kann mit Wasser verdünnt sein; beispielsweise kann eine 50 %ige Essigsäure eingesetzt werden.

Im Anschluß an die Zusammengabe der Reaktionskomponenten und der Carbonsäure läßt man die weiter oben formelmäßig beschriebene Reaktion im Verlaufe einer Zeit von 0,5 bis 24 Stunden, bevorzugt 1 bis 15 Stunden ablaufen. Die Carbonsäure und das eliminierte Amin werden sodann durch wäßrige Aufarbeitung vom offenkettigen Zwischenprodukt (4-R¹-5-amino-penta-2,4-dien-nitril) getrennt. Hierbei fällt dieses Zwischenprodukt, das in Wasser schwer löslich ist, in Substanz an oder es wird durch Extraktion mit einem wasserunlöslichen Lösungsmittel als ein solcher Extrakt gewonnen. Diese wäßrige Aufarbeitung ergibt sich in sehr eleganter Weise auch dann, wenn zur Durchführung des ersten Reaktionsschrittes bereits in Gegenwart eines mit Wasser nicht mischbaren Lösungsmittels, beispielsweise in Gegenwart von halogenierten Kohlenwasserstoffen gearbeitet wird. Eine solche Arbeitsweise ist daher bevorzugt.

Die Ringschlußreaktion zum 2,5-disubstituierten Pyridin erfolgt mit 0,7 bis 12 Äquivalenten, bevorzugt 1 bis 6 Äquivalenten einer Protonensäure oder von Ammoniak. Diese Protonensäuren können beispielsweise Chlorwasserstoffsäure, Bromwasserstoffsäure, Schwefelsäure, Phosphorsäure, Sulfonsäure oder eine äquivalente starke Säure sein. Für den Fall des Einsatzes von Chlorwasserstoffsäure oder Bromwasserstoffsäure wird der in 2-Stellung befindliche Substituent X Chlor oder Brom. Für den Fall des Einsatzes der anderen genannten Protonensäuren wird X zu Hydroxy. Es ist sehr erstaunlich, daß anstelle einer Säure auch Ammoniak eingesetzt werden kann, wobei im Zuge der Ringschlußreaktion der Substituent X die Bedeutung von Amino annimmt.

Halogenwasserstoff bzw. Ammoniak, wenn sie gasförmig eingeleitet werden, können auch in einer Menge eingesetzt werden, die über die obigen Werte hinausgeht.

Die Ringschlußreaktion in Gegenwart einer Protonensäure oder in Gegenwart von Ammoniak findet in einem Reaktionsmedium statt, das aus C₁-C₈-Carbonsäuren, halogenierten Kohlenwasserstoffen, Alkoholen, Amiden, weiteren oben genannten, säurefesten Lösungsmitteln oder einem Gemisch daraus gebildet sein kann. In bevorzugter Weise wird in einem Gemisch aus Carbonsäuren und halogenierten Kohlenwasserstoffen gearbeitet. Das offenkettige Zwischenprodukt, die Protonensäure bzw. das Ammoniak und das Reaktionsmedium können in beliebiger Reihenfolge zusammengegeben werden; beim Ringschluß durch eine Protonensäure wird bevorzugt die Säure vorgelegt und das Zwischenprodukt zudosiert. Die Ringschlußreaktion erfolgt im allgemeinen im Zeitbereich von 0,1 bis 5 Stunden, wobei diese Zeit selbstverständlich abhängig von der Ansatzgröße ist.

### Beispiele

### A) Ausgangsverbindungen

### Beispiel A1

324,4 g 90 %iger Ethylaminalester (Rest DMF), 123 g Acetonitril und 300 ml DMF wurden in einem 1,3 l V4A-Autoklaven 8 h auf 130°C erhitzt. Das Rohprodukt wurde destilliert (80°C/0,3 mm), und man erhielt 187 g 99,3 %iges trans-3-Dimethylaminoacrylnitril (0,45 % DMF; cis:trans = 2:98 gemäß NMR-Spektrum), entsprechend 95,4 % der theoretischen Ausbeute.

### Beispiel A2

60 g Acetonitril, 60 g DMF-dimethylacetal, 36 g Pyrrolidin und 150 ml DMF wurden 8 h am Rückfluß erhitzt. Nach Abdestillieren flüchtiger Bestandteile erhielt man nahezu quantitativ in 96 %iger Reinheit ein 87:13-Gemisch aus 3-Pyrrolidino- bzw. 3-Dimethylaminoacrylnitril (GC, GC/MS).

### Beispiel A3

In 200 g Morpholin und 10 g Kaliumcarbonat tropfte man innerhalb von 40 min unter Kühlung (25°C) 62 g frisch destillierten Propionaldehyd und rührte 2 h bei 25 bis 30°C. Nach kurzem Einengen am Rotationsverdampfer filtrierte man die Suspension. Mit dem Filtrat wurde eine Vakuumdestillation durchgeführt (30 cm-Vigreux-Kolonne mit Destillationskopf); der Druck betrug anfangs 30 mbar, zum Ende hin 10 mbar. Eine Produktfraktion, die bei 58 bis 65°C überdestillierte, enthielt zu 71 % 1-Morpholinopropen und zu 12 % das entsprechende Aminal (85,8 g, entsprechend 50,7 % der theoretischen Ausbeute).

### Beispiel A4

Zu einem Gemisch aus 213 g Pyrrolidin und 213 g Kaliumcarbonat wurde bei 0°C 108 g frisch destillierter Butyraldehyd zugetropft. Man ließ über Nacht bei Raumtemperatur stehen, verdünnte mit 300 ml Toluol und filtrierte vom Rückstand. Die anschließende Vakuumdestillation bei 85 bis 90°C (80 mbar) ergab 102 g 97 %iges 1-Pyrrolidinobuten, entsprechend 63,7 % der theoretischen Ausbeute).

### B) Umsetzungen

### Beispiel B1

14,1 g Morpholinopropen aus A3 wurden in 50 ml Methylenchlorid vorgelegt und bei -35°C 7,7 g 3-Dimethylamino-acrylnitril aus A1 zugetropft. Man ließ langsam auf 0°C auftauen, hielt 2 h bei 0°C und tropfte bei Raumtemperatur (RT) 16 ml 50 %ige Essigsäure zu. Nach 2 h Rühren bei RT trennte man die Phasen und extrahierte die wäßrige Phase. Die eingeengten Extrakte wurden durch GC/MS untersucht und als Hauptprodukt wurden 4-Methyl-5-morpholino-penta-2,4-dien-nitril neben dem entsprechenden Dimethylaminoderivat identifiziert. Edukte und umaminierte Edukte waren noch zu 15 % vorhanden.

### Beispiel B2

13 g Pyrrolidinobuten aus A4 wurden in 50 ml Methylenchlorid vorgelegt und bei -30°C 11,9 g 3-Pyrrolidino-acrylnitril aus A2 zugetropft. Man arbeitete, wie in Beispiel B1 beschrieben, weiter und konnte durch GC/MS 4-(1-Pyrrolidino-methylen)-hexennitril-2 als Hauptprodukt identifizieren.

### Beispiel B3

Das Rohprodukt aus B1 wurde in einem Gemisch aus 80 ml Essigsäure und 20 ml Chloroform gelöst, und bei 0°C wurde 30 min lang trockenes Chlorwasserstoffgas eingeleitet. Man rührte noch 1 h bei 0°C und 4 h bei 25°C. Nach dem Einengen und der Zugabe von Toluol und Wasser wurde mit Natriumcarbonat auf pH 7-8 gestellt. Die organische Phase wurde abgetrennt, eingeengt und mit Hilfe einer Kugelrohrdestillation 2-Chlor-5-methylpyridin in 37,5 % der theoretischen Ausbeute erhalten. Das Produkt wurde durch GC- und GC/MS-Vergleich mit authentischer Ware identifiziert. H-NMR (CDCl₃): 7,22 (H³); 7,48 (H⁴); 8,62 (H⁶) ppm.

### Beispiel B4

Mit dem Rohprodukt aus B2 wurde analog zu B3 verfahren. 2-Chlor-5-ethyl-pyridin wurde in 39,6 % erhalten und durch GC/MS-Kopplung und H-NMR identifiziert.

### Beispiel B5

Rohes 4-Methyl-5-morpholino-pentadien-2,4, das analog Beispiel 1 hergestellt wurde, versetzte man mit 20 ml konzentrierter Schwefelsäure und goß anschließend auf Eis. Als Reaktionsprodukt wurde hauptsächlich 2-Hydroxy-5-methylpyridin erhalten.

### Beispiel B6

30 g Pyrrolidino-buten aus A4 wurden in 50 ml Chloroform bei -10°C mit 19,3 g β-Pyrrolidino-acrylnitril versetzt. Man ließ auf RT kommen und tropfte 50 ml Essigsäure zu. Nach 15 h Rühren bei RT wurde auf -10°C abgekühlt und trockenes Chlorwasserstoffgas eingeleitet. Nach wäßriger Aufarbeitung wurde 2-Chlor-5-ethylpyridin als Hauptprodukt gewonnen.

## Patentansprüche

1. Verfahren zur Herstellung von 2,5-disubstituierten Pyridinen der Formel 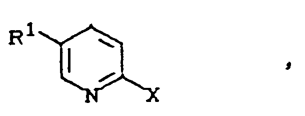 gekennzeichnet durch die Umsetzung von 0,2-5 Mol, bevorzugt 0,4-3 Mol, besonders bevorzugt 0,7-1,5 Mol eines Enamins der Formel 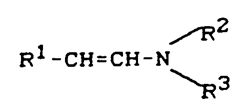 mit 1 Mol eines β-Amino-acrylnitrils der Formel 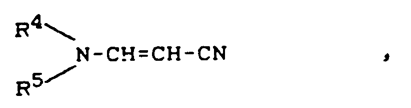 wobei in den Formeln
R¹, R², R³, R⁴ und R⁵ unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ bzw. R⁴ und R⁵ jeweils gemeinsam, jedoch unabhängig voneinander, mit dem N-Atom, das sie substituieren, einen 5-bis 8-gliedrigen Ring bilden können, der ein weiteres Heteroatom aus der Gruppe von N, O und S enthalten kann, und
X für Chlor, Brom, Hydroxy oder Amino steht,
im Temperaturbereich von -70°C bis +50°C, bevorzugt -40°C bis +25°C, in flüssiger Phase und in Gegenwart von 0,7-20 Äquivalenten, bevorzugt 1-10 Äquivalenten, einer C₁-C₈-Carbonsäure zu einem offenkettigen Zwischenprodukt und anschließender Ringschlußreaktion mit 0,7-12 Äquivalenten, bevorzugt 1-6 Äquivalenten, einer Protonensäure oder von Ammoniak in einem Reaktionsmedium aus C₁-C₈-Carbonsäuren, halogenierten Kohlenwasserstoffen, Alkoholen, Amiden oder einem Gemisch daraus.

2. Verfahren nach Anspruch 1, dadurch gekennzeichnet, daß an die Stelle von R¹ der Substituent R¹¹ tritt, der geradkettiges oder verzweigtes C₁-C₈-Alkyl, Cyclopropyl, Cyclopentyl, Cyclohexyl, Phenyl oder Benzyl bedeutet.

3. Verfahren nach Anspruch 2, dadurch gekennzeichnet, daß an die Stelle von R¹¹ der Substituent R²¹ tritt, der geradkettiges oder verzweigtes C₁-C₄-Alkyl bedeutet.

4. Verfahren nach Anspruch 1, dadurch gekennzeichnet, daß an die Stelle von R², R³, R⁴ und R⁵ die Substituenten R¹², R¹³, R¹⁴ und R¹⁵ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ bzw. R¹⁴ und R¹⁵ jeweils gemeinsam, jedoch unabhängig voneinander, mit dem N-Atom, das sie substituieren, einen 5- bis 8-gliedrigen Ring bilden können, der ein weiteres Heteroatom aus der Gruppe von N, O und S enthalten kann.

5. Verfahren nach Anspruch 4, dadurch gekennzeichnet, daß an die Stelle von R¹², R¹³, R¹⁴ und R¹⁵ die Substituenten R²², R²³, R²⁴ und R²⁵ treten, die unabhängig voneinander geradkettiges oder verzweigtes C₁-C₄-Alkyl bedeuten, wobei weiterhin R²² und R²³ bzw. R²⁴ und R²⁵ jeweils gemeinsam, jedoch unabhängig voneinander, mit dem N-Atom, das sie substituieren, Morpholino, Pyrrolidin oder Piperidin, das durch C₁-C₄-Alkyl oder durch Hydroxy-C₁-C₄-alkyl substituiert sein kann, bedeuten können.

6. Verfahren nach Anspruch 1, dadurch gekennzeichnet, daß als Carbonsäure für die Reaktion zum offenkettigen Zwischenprodukt Essigsäure oder Propionsäure, bevorzugt Essigsäure, eingesetzt wird.

7. Verfahren nach Anspruch 1, dadurch gekennzeichnet, daß zur Einstellung der flüssigen Phase für die Reaktion zum offenkettigen Zwischenprodukt ein Lösungsmittel aus der Gruppe der halogenierten Kohlenwasserstoffe, der Ketone, der Nitrile, der Amide, der Ester und der Ether oder einem Gemisch mehrerer von ihnen eingesetzt wird.

8. Verfahren nach Anspruch 7, dadurch gekennzeichnet, daß ein oder mehrere halogenierte Kohlenwasserstoffe eingesetzt werden.

9. Verfahren nach Anspruch 1, dadurch gekennzeichnet, daß die Ringschlußreaktion in einem Gemisch aus Carbonsäure und halogeniertem Kohlenwasserstoff durchgeführt wird.

## Claims

1. Process for the preparation of 2,5-disubstituted pyridines of the formula 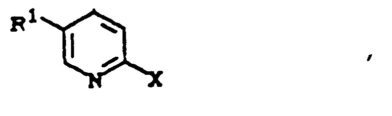 characterised by the reaction of 0.2-5 mol, preferably 0.4-3 mol, particularly preferably 0.7-1.5 mol, of an enamine of the formula 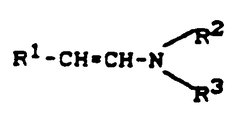 with 1 mol of a β-amino-acrylonitrile of the formula 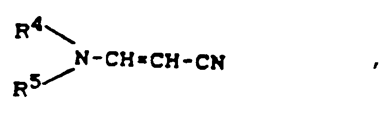 in which formulae
R¹, R², R³, R⁴ and R⁵, independently of one another, represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl or a 5- to 8-membered saturated or unsaturated heterocyclic ring whose hetero atoms are 1 or 2 from the group consisting of N, O and S, it furthermore being possible for R² and R³ or R⁴ and R⁵, in each case together, but independently of one another, with the N atom which they substitute, to form a 5- to 8-membered ring which may contain a further hetero atom from the group consisting of N, O and S, and
X represents chlorine, bromine, hydroxyl or amino,
in the temperature range of from -70°C to +50°C, preferably -40°C to +25°C, in liquid phase and in the presence of 0.7-20 equivalents, preferably 1-10 equivalents, of a C₁-C₈-carboxylic acid to give an open-chain intermediate, followed by a ring-closure reaction with 0.7-12 equivalents, preferably 1-6 equivalents, of a proton acid or of ammonia in a reaction medium consisting of C₁-C₈-carboxylic acids, halogenated hydrocarbons, alcohols, amides or a mixture thereof.

2. Process according to Claim 1, characterised in that the substituent R¹¹, which denotes straight-chain or branched C₁-C₈-alkyl, cyclopropyl, cyclopentyl, cyclohexyl, phenyl or benzyl, takes the place of R¹.

3. Process according to Claim 2, characterised in that the substituent R²¹, which denotes straight-chain or branched C₁-C₄-alkyl, takes the place of R¹¹.

4. Process according to Claim 1, characterised in that the place of R², R³, R⁴ and R⁵ is taken by substituents R¹², R¹³, R¹⁴ and R¹⁵ which, independently of one another, denote straight-chain or branched C₁-C₈-alkyl, cyclopropyl, cyclopentyl, cyclohexyl, phenyl or benzyl, it furthermore being possible for R¹² and R¹³ or R¹⁴ and R¹⁵, in each case together, but independently of one another, with the N atom which they substitute, to form a 5- to 8-membered ring which may contain a further hetero atom from the group consisting of N, O and S.

5. Process according to Claim 4, characterised in that the place of R¹², R¹³, R¹⁴ and R¹⁵ is taken by the substituents R²², R²³, R²⁴ and R²⁵ which, independently of one another, denote straight-chain or branched C₁-C₄-alkyl, it furthermore being possible for R²² and R²³ or R²⁴ and R²⁵, in each case together, but independently of one another, with the N atom which they substitute, to denote morpholino, pyrrolidine or piperidine, which may be substituted by C₁-C₄-alkyl or by hydroxy-C₁-C₄-alkyl.

6. Process according to Claim 1, characterised in that acetic acid or propionic acid, preferably acetic acid, is used as the carboxylic acid for the reaction to give the open-chain intermediate.

7. Process according to Claim 1, characterised in that a solvent from the group of the halogenated hydrocarbons, the ketones, the nitriles, the amides, the esters and the ethers or a mixture of a plurality thereof is used for adjusting the liquid phase for the reaction to give the open-chain intermediate.

8. Process according to Claim 7, characterised in that one or more halogenated hydrocarbons are used.

9. Process according to Claim 1, characterised in that the ring-closure reaction is carried out in a mixture of carboxylic acid and halogenated hydrocarbon.

## Revendications

1. Procédé de préparation de pyridines 2,5-disubstituées de formule : 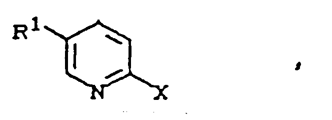 caractérisé en ce que l'on fait réagir de 0,2 à 5 mol, de préférence de 0,4 à 3 mol et plus spécialement de 0,7 à 1,5 mol d'une énamine de formule : 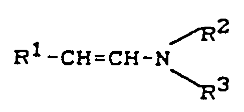 avec 1 mol d'un β-aminoacrylonitrile de formule : 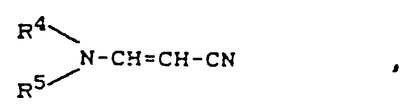 les symboles de ces formules ayant les significations suivantes :
R¹, R², R³, R⁴ et R⁵ représentent chacun, indépendamment les uns des autres, un groupe alkyle en C₁-C₈, alcényle en C₂-C₈, alcoxyalkyle en C₂-C₈, alcoxyalcényle en C₃-C₈ à chaîne droite ou ramifiée, cycloalkyle en C₃-C₈, aryle en C₆-C₁₂, aralkyle en C₇-C₁₀ ou un hétérocycle saturé ou insaturé de 5 à 8 chaînons contenant un ou deux hétéroatomes choisis parmi N, O et S, R² et R³ d'une part, R⁴ et R⁵ d'autre part, pouvant former ensemble, indépendamment, et avec l'atome d'azote auquel ils sont reliés, un cycle de 5 à 8 chaînons qui peut contenir un autre hétéroatome choisi parmi N, O et S, et
X représente le chlore, le brome, un groupe hydroxy ou amino,
dans l'intervalle de température de -70 à +50°C, de préférence de -40 à +25°C, en phase liquide et en présence de 0,7 à 20 équivalents, de préférence de 1 à 10 équivalents d'un acide carboxylique en C₁-C₈, la réaction donnant un produit intermédiaire acyclique qu'on soumet ensuite à une réaction de cyclisation à l'aide de 0,7 à 12 équivalents, de préférence 1 à 6 équivalents d'un acide protonique ou d'ammoniac dans un milieu de réaction consistant en acides carboxyliques en C₁-C₈, hydrocarbures halogénés, alcools, amides ou leurs mélanges.

2. Procédé selon la revendication 1, caractérisé en ce que, à la place du substituant R¹, il y a un substituant R¹¹ qui consiste en un groupe alkyle en C₁-C₈ à chaîne droite ou ramifiée, cyclopropyle, cyclopentyle, cyclohexyle, phényle ou benzyle.

3. Procédé selon la revendication 2, caractérisé en ce que, à la place du substituant R¹¹, il y a un substituant R²¹ qui consiste en un groupe alkyle en C₁-C₄ à chaîne droite ou ramifiée.

4. Procédé selon la revendication 1, caractérisé en ce que, à la place des substituants R², R³, R⁴ et R⁵, il y a les substituants R¹², R¹³, R¹⁴ et R¹⁵ qui représentent chacun, indépendamment les uns des autres, un groupe alkyle en C₁-C₈ à chaîne droite ou ramifiée, cyclopropyle, cyclopentyle, cyclohexyle, phényle ou benzyle, R¹² et R¹³ d'une part, R¹⁴ et R¹⁵ d'autre part, pouvant également former ensemble, mais indépendamment, et avec l'atome d'azote auquel ils sont reliés, un cycle de 5 à 6 chaînons qui peut contenir un autre hétéroatome choisi parmi N, O et S.

5. Procédé selon la revendication 4, caractérisé en ce que, à la place de R¹², R¹³, R¹⁴ et R¹⁵, il y a les substituants R²², R²³, R²⁴ et R²⁵ qui consistent chacun, indépendamment les uns des autres, en un groupe alkyle à chaîne droite ou ramifiée en C₁-C₄, R²² et R²³ d'une part, R²⁴ et R²⁵ d'autre part, pouvant également former ensemble, mais indépendamment, et avec l'atome d'azote auquel ils sont reliés, un cycle morpholino, pyrrolidino ou pipéridino qui peut être substitué par un groupe alkyle en C₁-C₄ ou hydroxyalkyle en C₁-C₄.

6. Procédé selon la revendication 1, caractérisé en ce que l'acide carboxylique utilisé pour la réaction conduisant au produit intermédiaire acyclique est l'acide acétique ou l'acide propionique, de préférence le premier nommé.

7. Procédé selon la revendication 1, caractérisé en ce que, pour former une phase liquide dans la réaction conduisant au produit intermédiaire acyclique, on utilise un solvant choisi parmi les hydrocarbures halogénés, les cétones, les nitriles, les amides, les esters et les éthers ou un mélange de plusieurs d'entre eux.

8. Procédé selon la revendication 7, caractérisé en ce que l'on utilise un ou plusieurs hydrocarbures halogénés.

9. Procédé selon la revendication 1, caractérisé en ce que la réaction de cyclisation est réalisée dans un mélange d'un acide carboxylique et d'un hydrocarbure halogéné.
